Europäisches Patentamt

(19) European Patent Office

Office européen des brevets

(11) Publication number: **0 072 541**
**A2**

(12) # EUROPEAN PATENT APPLICATION

(21) Application number: 82107337.6

(22) Date of filing: 12.08.82

(51) Int. Cl.³: **C 12 N 15/00**
C 07 C 103/52, C 12 P 21/02
C 12 N 1/20, A 61 K 45/02
C 07 H 21/04
//C12R1/19

(30) Priority: 14.08.81 US 293044

(43) Date of publication of application:
23.02.83 Bulletin 83/8

(84) Designated Contracting States:
AT BE CH DE FR GB IT LI NL

(71) Applicant: F. HOFFMANN-LA ROCHE & CO.
Aktiengesellschaft

CH-4002 Basel(CH)

(71) Applicant: GENENTECH, INC.
460 Point San Bruno Boulevard
So. San Francisco California 94080(US)

(72) Inventor: Van Goeddel, David
1449 Benito Avenue
Burlingame, CA(US)

(72) Inventor: Pestka, Sidney
82 Brookside Terrace
North Caldwell, N.J.(US)

(74) Representative: Lederer, Franz, Dr. et al,
Patentanwälte Dr. Lederer Franz Meyer-Roxlau Reiner F.
Lucile-Grahn-Strasse 22
D-8000 München 80(DE)

(54) Human leukocyte interferons, process for their microbial production, intermediates therefor and compositions containing them.

(57) Mature human leukocyte interferon species K and L, useful in the treatment of viral and neoplastic diseases, and their preparation by recombinant DNA technology as well as the means used in this process, i.e. recombinant DNA molecules coding for the amino acid sequences of said interferons, vectors capable of expressing said interferons in microbial host organisms and host organisms transformed with these vectors.

Figure 1

EP 0 072 541 A2

Croydon Printing Company Ltd.

F. Hoffmann-La Roche & Co.    GENENTECH, Inc.        0072541
Aktiengesellschaft       and  South San Francisco
Basel / Schweiz               Ca., USA          RAN 4100/19

— 1 —

## Human leukocyte interferons, process for their microbial production, intermediates therefor and compositions containing them

The present invention relates to the field of recombinant DNA technology, i.e. to processes used in recombinant DNA technology and to products obtained by these processes.

In a more detailed aspect the present invention relates to polypeptides, specifically to mature human leukocyte interferons K and L, to pharmaceutical compositions containing them and to a process for their preparation which comprises causing a culture of a microorganism transformed with a replicable microbial expression vehicle capable of expressing said polypeptides to grow up and express said polypeptides. The present invention also comprises the expression vehicles used in this process and the novel microorganisms containing these expression vehicles as well as the processes for their preparation. Finally, the invention relates to DNA sequences comprising sequences coding for the amino acid sequences of mature human leukocyte interferons K and L.

Human leukocyte interferon (LeIF) species A through J a process for their microbial production (i.e. using recombinant DNA technology), intermediates in this process (DNA sequences, replicable microbial expression vehicles, e.g. plasmids, and transformed microorganisms) and pharmaceutical compositions containing them are disclosed and claimed e.g. in European published patent application No. 43 980 and Japanese published patent application No. 79897/82 of applicants. The direct expression of mature leukocyte interferons and the structure of eight distinct cloned human leukocyte interferon cDNAs as well as the

Mez/8.7.82

corresponding amino acid sequences of interferon species LeIF-A through H have been described by Goeddel et al. in Nature 287, 411 (October 2, 1980) and Nature 290, 20 (March 5, 1981).

The present invention-using the methods described in detail in the above-mentioned patent specifications and in the Nature articles (which are all incorporated into the present specification by reference) which have mean while become methods well known in the art - extends the family of recombinant human leukocyte interferon species by two additional species, K and L. More specificly, identification of human leukocyte interferon K and L genes has, through recombinant DNA technology, enabled the microbial production of these additional members of the family of homologous leukocyte interferons (sans glycosylation) as mature polypeptides,i.e. unaccompanied by the corresponding presequence or any portion thereof. They may be directly expressed, recovered and purified to levels fitting for use in the treatment of viral and malignant diseases of animals and man.

Reference herein to the expression of a "mature leukocyte interferon," connotes the bacterial or other microbial production of an interferon molecule unaccompanied by associated glycosylation and the presequence that immediately attends mRNA translation of a human leukocyte interferon genome. Mature leukocyte interferon, according to the present invention, is immediately expressed from a translation start signal (ATC) just before the first amino acid codon of the natural product, in which event the mature polypeptide includes the methionine for which ATG codes without essentially altering its character, or the microbial host may process the translation product to delete the initial methionine.

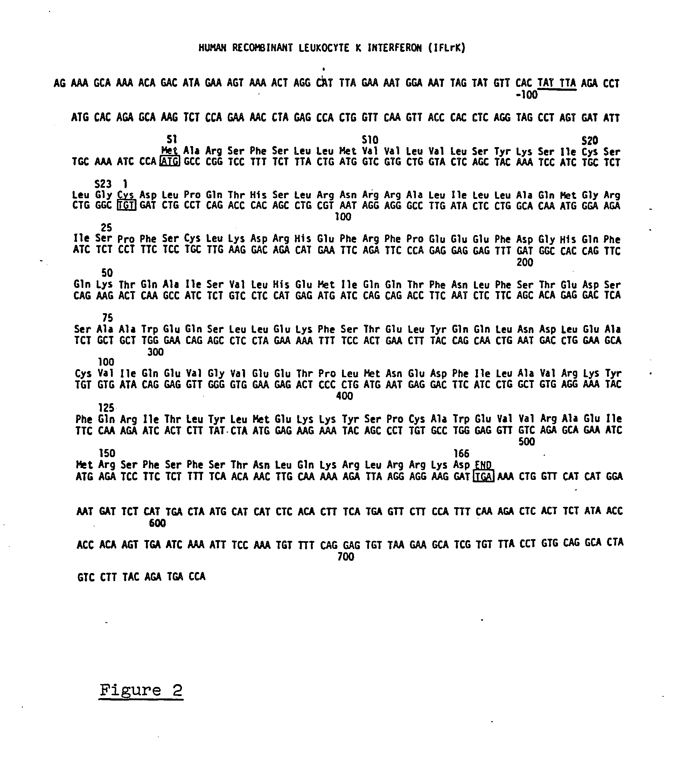

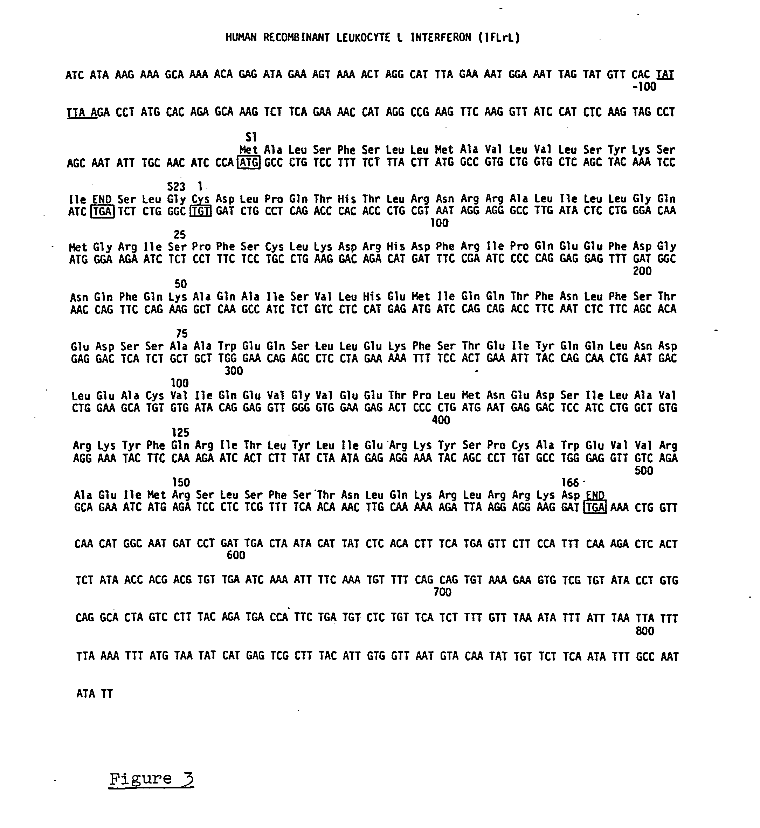

The novel leukocyte interferon proteins identified as LeIF K and LeIF L have been defined by means of determined DNA sequence of the gene and deductive amino acid sequencing (see Figures 2 and 3). It will be understood that for these particular interferons, natural allelic variations exist and occur from individual to individual. These variations may be demonstrated by (an) amino acid difference(s) in the overall sequence or by deletions, substitutions, insertions, inversions or additions of (an) amino acid(s) in said sequence. For the leukocyte interferon protein hereof, such allelic variations are included within the scope of the label or term defining such, and thus, this invention.

Brief description of the drawings

Figure 1   shows the restriction enzyme cleavage site on the 14-kb insert of genomic DNA clone G8.

Figure 2   provides the DNA and corresponding amino acid sequences of LeIF-K.

Figure 3   provides the DNA and corresponding amino acid sequences of LeIF-L.

Figures 4 and 5 show the construction of the recombinant plasmids pLeIFrK and pLeIFrL respectively.

DESCRIPTION OF THE PREFERRED EMBODIMENTS

A.   Microorganisms employed

The work described involved use of E. coli K-12 strain 294 (end A, thi$^-$, hsr$^-$, hsm$_k^+$), as described in British patent publication No. 2055382A and deposited with the American Type Culture Collection, ATCC accession No. 31446. All recombinant DNA work was performed in compliance with applicable guidelines of the National Institutes of Health.

The invention, in its most preferred embodiments, is described with reference to E. coli, including not only strain E. coli K-12 strain 294, defined above, but also other known E. coli strains such as E. coli x 1776, E. coli B, or other microbial strains deposited with and available from recognized microorganism depository institutions, such as the American Type Culture Collection (ATCC). These other microorganisms include, for example, bacilli such as Bacillus subtilis and other enterobacteriaceae among which can be mentioned as examples Salmonella typhimurium and Serratia marcescens, utilizing plasmids that can replicate and express heterologous gene sequences therein. Yeast, such as Saccharomyces cerevisiae, may also be employed to advantage as host organism in the preparation of the interferon proteins hereof by expression of genes coding therefore under the control of a yeast promotor.

B.    LeIF-K and -L

The phage λ Charon 4A recombinant library of the human genome constructed by Lawn et al., Cell 15, 1157 (1978), was screened for additional leukocyte interferon (LeIF) genes by in situ plaque hybridization (Benton and Davis, Science 196, 180 [1977]). A radioactive LeIF probe derived from the cDNA clone LeIF-A (Goeddel et al., Nature 287, 411 [1980]) was used and provided a further position clone which appeared distinct. This clone (G8) provided the following EcoRI fragments: 3.3, 3.1, 2.9, 2.2, 1.4 and 1.3 kb pairs of which the 2.2, 1.4 and 1.3 kb fragments hybridized to the radiolabeled cDNA probe after separation by 0.8% agarose gel electrophoresis followed by transfer to nitro cellulose filter paper.

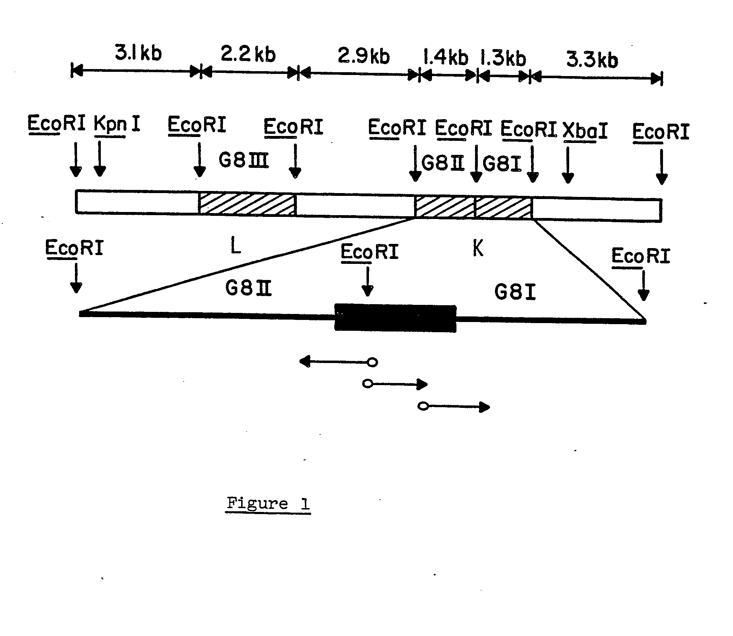

Figure 1 shows a map of restriction enzyme cleavage sites on the 14.2 kb insert of genomic DNA clone G8. The three fragments hybridizing to the leukocyte interferon cDNA were sequenced with internal primers obtained from plasmid 104 containing interferon cDNA sequences (Maeda et al., Proc. Natl. Acad. Sci. U.S.A. <u>77</u>, 7010 [1980]) or to a synthetic oligonucleotide primer complementary to the M13 DNA region preceding the EcoRI site as shown in Figure 1.

In Figure 1 vertical arrows indicate the restriction enzyme cleavage sites. Hatched boxes represent the EcoRI fragments hybridizing to the cDNA probe. A solid box represents the protein-coding region. Horizontal arrows represent the direction and length of dideoxynucleotide termination sequencing reaction initiating from the primers indicated by open circles.

The total DNA sequence of the human LeIF K and L genes are shown in Figures 2 and 3 respectively. The amino acid sequences are presented above the nucleotide sequences.

## PROTOCOL FOR EXPRESSION OF LeIF-rK

The following fragments are isolated to construct an expression plasmid for LeIF-rK:

(a) An approximate 300 b.p. fragment (HindIII-Sau3A) containing the trp operator promoter, ATG start codon and cysteine codon of LeIF-rA (pLeIF-A25).

(b) An approximate 99 b.p. fragment (EcoRI-Sau3A) from the fragment designated λ G8-II (1.4 kb) containing the amino-terminal coding portion of LeIF-G8-I (a genomic DNA isolated from the human gene library).

(c) The fragment designated λ G8-I of approximate 1.3 kb containing the carboxy-terminal coding portion of LeIF-G8-I (EcoRI-EcoRI).

(d) An EcoRI-HindIII fragment from pBR322 (4.3 kb) containing the replicon and coding region
for ampicillin and tetracycline resistance.

Step 1:

Fragment (a) is ligated to fragment (d) to yield
a 4.6 kb linear fragment (Sau3A-EcoRI). This is ligated to fragment (b) to yield a circular molecule. The
above ligations are performed simultaneously as a 3-piece
ligation. Transformants are selected for ampicillin and
tetracycline resistance, screened by restriction mapping
and a clone is selected for further work. The correct molecule has 3 EcoRI sites.

Step 2:

The plasmid from Step 1 is partially digested with
EcoRI and the linear form is isolated by gel electrophoresis. The linear EcoRI fragment is ligated to fragment
(c). With 3 sites of insertion and 2 orientations possible,
one-sixth of the resultant transformants are in the proper
configuration to produce leukocyte interferon K (LeIF-rK).

Summary of the Recombinant Plasmid pLeIF-rK:

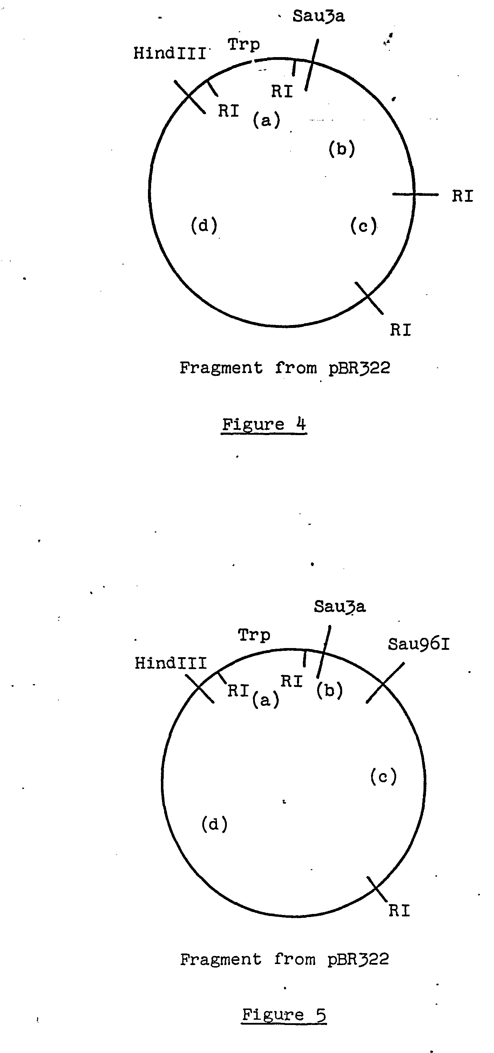

The recombinant plasmid, pLeIF-rK, was constructed
as described above. Upon transformation into E. coli K-12,
this plasmid produced a protein of 166 amino acids equivalent to one of the species of human leukocyte interferon
in addition to $NH_2$-terminal methionine (initiation codon).
The plasmid contains the fragments indicated in Figure 4.

The interferon DNA sequence codes for a protein consistent with protein sequence data derived from purified human
leukocyte interferons (Rubinstein et al., Arch. Biochem.
Biphys. 210, 307-318 [1981]; Levy et al., Proc. Natl.
Acad. Sci. U.S.A. 78, 6186-6190 [1981]; Levy et al., Proc.
Natl. Acad. Sci. U.S.A. 77, 5102-5104 [1980]; Zoon et al.,

Science 207, 527-528 [1980]). The remainder of the DNA contains 3'-untranslated portions of the gene, but contains no other genes.

## PROTOCOL FOR EXPRESSION OF LeIF-rL

The following fragments are isolated to construct an expression plasmid for LeIF-rL:

(a) A 35 b.p. Sau3a-Sau961 fragment from G8-III (2.2 kb).

(b) A Sau96I-EcoRI fragment to 3'-end of G8-III segment (about 1300 b.p.).

(c) An approximate 300 b.p. fragment (HindIII-Sau3a) from pLeIF-A25 as above.

(d) An EcoRI-HindIII-fragment (4.3 kb) from pBR322 as above.

## Step 1:

Fragment (a) is ligated to fragment (b) and (c) in three-part ligation mixture; the resulting a-b-c segments are cleaved with HindIII and EcoRI to minimize polymerization. The isolated segment c-a-b is ligated to (d) to yield the expression plasmid. Transformants are selected for ampicillin and tetracycline resistance. Transformations are screened by restriction mapping. The correct molecule has a size of approximately 6000 b.p., contains three EcoRI sites and upon transformation into E. coli K12 produces a protein (LeIF-rL) of 166 amino acids that is equivalent to one of the species of human leukocyte interferon in addition to the $NH_2$-terminal methionine (initiation codon). The plasmid contains the fragments indicated in Fig. 5.

The interferon DNA sequence codes for a protein consistent with protein sequence data derived from purified human leukocyte interferons (Rubinstein et al., Arch.
Biochem. Biophys. 210, 307-318 [1981]; Levy et al., Proc.
Natl. Acad. Sci. U.S.A. 78, 6186-6190 [1981]; Levy et al.,
Proc. Natl. Acad. Sci. U.S.A. 77, 5102-5104 [1980]; Zoon
et al., Science 207, 527-528 [1980]). The remainder of the
DNA contains 3'-untranslated portions of the gene, but
contains no other genes.

The contents of recombinant LeIF-K and -L in bacterial extracts may be enhanced and the compounds can be
obtained in purified form by using the procedures described in detail in European published patent application
No. 43980 or Japanese published patent application No.
79897/82.

LeIF-K and -L may be parenterally administered to subjects requiring antitumor or antiviral treatment, and to
those exhibiting immunosuppressive conditions. Dosage
and dose rate may parallel that currently in use in clinical investigations of human derived materials, e.g.,
about $(1-10) \times 10^6$ units daily, and in the case of materials of purity greater than 1%, likely up to, e.g.,
$5 \times 10^7$ units daily.

As one example of an appropriate dosage form for
essentially homogeneous bacterial LeIF in parenteral form,
3 mg LeIF of specific activity of, say, $2 \times 10^8$ units/mg
may be dissolved in 25 ml of 5 N human serum albumin, the
solution is passed through a bacteriological filter and the
filtered solution aseptically subdivided into 100 vials,
each containing $6 \times 10^6$ units pure interferon suitable for
parenteral administration. The vials are preferably stored
in the cold (-20°C) prior to use.

The interferon species of the present invention can be
formulated according to known methods to prepare pharmaceutically useful compositions, whereby the polypeptide hereof is combined in admixture with a pharmaceutically acceptable carrier vehicle. Suitable vehicles and their formulation are described in Remington's Pharmaceutical Sciences by E.W. Martin, which is hereby incorporated into the specification by reference. Such compositions will contain an effective amount of the interferon protein hereof together with a suitable amount of vehicle in order to prepare pharmaceutically acceptable compositions suitable for effective administration to the host. One preferred mode of administration is parenteral.

What we claim is:

1. A polypeptide comprising the amino acid sequence of mature human leukocyte interferon K or L, unaccompanied by any presequence.

2. A polypeptide according to claim 1, in unglycosylated form.

3. A polypeptide comprising the amino acid sequence of mature human leukocyte interferon K or L, containing the amino acid methionine attached to the N-terminus of the ordinarily first amino acid of said interferon.

4. Mature human leukocyte interferon K (LeIF-K).

5. Mature human leukocyte interferon L (LeIF-L).

6. A polypeptide according to any one of claims 1 to 5 microbially produced.

7. A polypeptide according to any one of claims 1 to 5 bacterially produced.

8. A polypeptide according to any one of claims 1 to 5 produced by E. coli.

9. A DNA sequence comprising the sequence coding for the amino acid sequence of mature human leukocyte interferon K or L unaccompanied by any presequence.

10. A DNA sequence comprising the sequence coding for the amino acid sequence of mature human leukocyte interferon K or L containing the amino acid methionine attached to the N-terminus of the ordinarily first amino acid of said interferon.

11. A DNA sequence coding for mature human leukocyte interferon K (LeIF-K).

12. A DNA sequence coding for mature human leukocyte interferon L (LeIF-L).

13. A DNA sequence according to any one of claims 9 to 12 operably linked with a DNA sequence capable of effecting microbial expression of a polypeptide according to any one of claims 1 to 8.

14. A DNA sequence according to any one of claims 9 to 12 operably linked with a DNA sequence capable of effecting expression of a polypeptide according to any one of claims 1 to 8 in a bacterium.

15. A DNA sequence according to any one of claims 9 to 12 operably linked with a DNA sequence capable of effecting expression of a polypeptide according to any one of claims 1 to 8 in E. coli.

16. A replicable microbial expression vehicle capable, in a transformant microorganism, of expressing a polypeptide according to any one of claims 1 to 8.

17. A replicable bacterial expression vehicle capable, in a transformant bacterium, of expressing a polypeptide according to any one of claims 1 to 8.

18. A replicable E. coli expression vehicle capable, in a transformant E. coli, of expressing a polypeptide according to any one of claims 1 to 8.

19. A microbial expression vehicle according to any one of claims 16-18 which is a plasmid.

20. A microorganism transformed with a vehicle as claimed in any one of claims 16 to 19.

21. A bacterium transformed with a vehicle as claimed in any one of claims 16 to 19.

22. A strain of E. coli transformed with a vehicle as claimed in any one of claims 16 to 19.

23. E. coli K-12 strain 294 transformed with a vehicle as claimed in any one of claims 16 to 19.

24. A composition of matter comprising a therapeutically active fraction of a polypeptide consisting essentially of the amino acid sequence of mature human leukocyte interferon K or L as claimed in any one of claims 1 to 5, the balance of said composition comprising soluble microbial protein from which said polypeptide may be purified to a degree sufficient for effective therapeutic application.

25. A bacterial extract comprising greater than about 95 percent pure polypeptide consisting essentially of the amino acid sequence of mature human leukocyte interferon K or L as claimed in any one of claims 1 to 5.

26. A pharmaceutical composition containing a therapeutically effective amount of mature human leukocyte interferon K or L as claimed in any one of claims 1 to 5 and a carrier material suitable for pharmaceutical administration.

27. A pharmaceutical composition as claimed in claim 26 for parenteral administration.

28. A culture of microbial cells capable of producing mature human leukocyte interferon K or L.

29. The culture of claim 28, which is a culture of a microorganism claimed in any one of claims 20 to 23.

ES 0072541

- 13 -

30. The use of the compounds claimed in any one of claims 1 to 8 for treatment of tumors and viral infections or for preparing pharmaceutical compositions useful for such treatment.

31. The use of the DNA sequences claimed in any one of claims 9 to 15 in the microbial production of a compound claimed in any one of claims 1 to 8.

32. The use of an expression vehicle as claimed in any one of claims 16 to 19 in the microbial production of a compound claimed in any one of claims 1 to 8.

33. The use of a microorganism as claimed in any one of claims 20 to 23 or of a culture of microbial cells as claimed in claims 28 and 29 in the production of a compound claimed in any one of claims 1 to 8.

34. The compounds claimed in any one of claims 1 to 8 whenever used for the treatment of tumors and viral infections or for preparing pharmaceutical compositions useful for such treatment.

35. The DNA sequences claimed in any one of claims 9 to 15 whenever used in the microbial production of a compound claimed in any one of claims 1 to 8.

36. An expression vehicle as claimed in any one of claims 16 to 19 whenever used in the microbial production of a compound claimed in any one of claims 1 to 8.

37. A microorganism as claimed in any one of claims 20 to 23 or a culture of microbial cells as claimed in claims 28 and 29 whenever used in the production of a compound claimed in any one of claims 1 to 8.

38. A process for preparing a polypeptide claimed in any one of claims 1 to 8 which process comprises causing a culture of a microorganism, transformed with a replicable microbial expression vehicle capable of expressing said polypeptide, to grow up and express said polypeptide and recovering said polypeptide.

39. A process for preparing microorganisms capable of expressing a polypeptide claimed in any one of claims 1 to 8 which process comprises transforming a microorganism with a replicable microbial expression vehicle capable of expressing said polypeptide and cultivating the transformed microorganism.

40. A process for preparing a replicable microbial expression vehicle capable, in a transformant microorganism of expressing a polypeptide as claimed in any one of claims 1 to 8, which process comprises constructing a first DNA sequence coding for said polypeptide and operably linking said first DNA sequence with a second DNA sequence capable of effecting microbial expression of said polypeptide.

***

AUSTRIA

What we claim is:

1. A process for the preparation of a polypeptide comprising the amino acid sequence of mature human leukocyte interferon K or L unaccompanied by any presequence, which process comprises causing a culture of a microorganism, transformed with a replicable microbial expression vehicle capable of expressing said polypeptide, to grow and express said polypeptide and recovering said polypeptide.

2. A process as claimed in claim 1, wherein the polypeptide is unglycosylated.

3. A process as claimed in claim 1, wherein the polypeptide comprises the amino acid sequence of mature human leukocyte interferon K or L and the amino acid methionine attached to the N-terminus of the ordinarily first amino acid of said interferon.

4. A process as claimed in claim 1 for the preparation of mature human leukocyte interferon K (LeIF-K).

5. A process as claimed in claim 1 for the preparation of mature human leukocyte interferon L (LeIF-L).

6. A process as claimed in any one of claims 1 to 5 wherein the microorganism is a bacterium.

7. A process as claimed in claim 6 wherein the bacterium is E. coli.

8. A process for the preparation of microorganisms capable of producing a polypeptide comprising the amino acid sequence of mature human leukocyte interferon K or L unaccompanied by any presequence which process comprises transforming a microorganism with a replicable microbial expression vehicle capable of expressing said polypeptide and cultivating the transformed microorganism.

9. A process as claimed in claim 8, wherein the expression vehicle is capable of expressing a polypeptide comprising the amino acid sequence of mature human leukocyte interferon k or L and the amino acid methionine attached to the N-terminus of the ordinarily first amino acid of said interferon.

10. A process as claimed in claim 8, wherein the expression vehicle is capable of expressing mature human leukocyte interferon K (LeIF-K).

11. A process as claimed in claim 8, wherein the expression vehicle is capable of expressing mature human leukocyte interferon L (LeIF-L).

12. A process as claimed in any one of claims 8 to 11 wherein the microorganism is a bacterium.

13. A process as claimed in claim 12 wherein the bacterium is E. coli.

14. A process for the preparation of a replicable expression vehicle capable, in a transformant microorganism, of expressing a polypeptide comprising the amino acid sequence of mature human leukocyte interferon K or L unaccompanied by any presequence, which process comprises constructing a first DNA sequence coding for said polypeptide and operably linking said first DNA sequence with a second DNA sequence capable of effecting microbial expression of said polypeptide.

15. A process for the preparation of a replicable bacterial expression vehicle in accordance with claim 14, characterized in that the second sequence is capable of effecting expression in a bacterium.

16. A process as claimed in claim 15 wherein the bacterium is E. coli.

17. A process as claimed in any one of claims 14 to 16, wherein the polypeptide comprises the amino acid sequence of a mature human leukocyte interferon and the amino acid methionine attached to the N-terminus of the ordinarily first amino acid of said interferon.

18. A process as claimed in any one of claims 14 to 16, wherein human leukocyte interferon K (LeIF-K) is expressed.

19. A process as claimed in any one of claims 14 to 16, wherein human leukocyte interferon L (LeIF-L) is expressed.

20. A process for the preparation of pharmaceutical compositions containing a microbially produced polypeptide comprising the amino acid sequence of human leukocyte interferon K or L which process comprises mixing said polypeptide with non-toxic, inert, therapeutically compatible carriers and bringing the resulting mixture into a suitable pharmaceutical dosage form.

21. A pharmaceutical composition containing a microbially produced polypeptide comprising the amino acid sequence of human leukocyte interferon K or L and a non-toxic, inert, therapeutically compatible carrier material.

22. A DNA sequence comprising the sequence coding for the amino acid sequence of mature human leukocyte interferon K or L unaccompanied by any presequence.

23. A DNA sequence comprising the sequence coding for the amino acid sequence of mature human leukocyte interferon K or L containing the amino acid methionine attached to the N-terminus of the ordinarily first amino acid of said interferon.

24. A DNA sequence coding for mature human leukocyte interferon K (LeIF-K).

25. A DNA sequence coding for mature human leukocyte interferon L (LeIF-L).

26. A DNA sequence according to any of claims 22 to 25 operably linked with a DNA sequence capable of effecting microbial expression of said polypeptide.

27. A DNA sequence according to any one of claims 22 to 25 operably linked with a DNA sequence capable of effecting expression of said polypeptide in a bacterium.

28. A DNA sequence as claimed in claim 27, wherein the bacterium is E. coli.

29. A replicable microbial expression vehicle capable, in a transformant microorganism, of expressing the polypeptides coded by the DNA sequences according to claims 22 to 28.

30. An expression vehicle as claimed in claim 29 capable of expressing the polypeptides in a bacterium.

31. An expression vehicle as claimed in claim 30, wherein the bacterium is E. coli.

32. A microbial expression vehicle according to any one of claims 29 to 31 which is a plasmid.

33. A microorganism transformed with a vehicle as claimed in any one of claims 29 to 32.

34. A bacterium transformed with a vehicle as claimed in any one of claims 29 to 32.

0072541

35. A strain of E. coli transformed with a vehicle as claimed in any one of claims 29 to 32.

36. E. coli K-12 strain 294 transformed with a vehicle as claimed in any one of claims 29 to 32.

***

Figure 1

RAN 4100/19

HUMAN RECOMBINANT LEUKOCYTE K INTERFERON (IFLrK)

```
AG AAA GCA AAA ACA GAC ATA GAA AGT AAA ACT AGG CAT TTA GAA AAT GGA AAT TAG TAT GTT CAC TAT TTA AGA CCT
                                                                                        -100

  ATG CAC AGA GCA AAG TCT CCA GAA AAC CTA GAG CCA CTG GTT CAA GTT ACC CAC CTC AGG TAG CCT AGT GAT ATT

              S1                                    S10                                   S20
              Met Ala Arg Ser Phe Ser Leu Leu Met Val Val Leu Val Leu Ser Tyr Lys Ser Ile Cys Ser
  TGC AAA ATC CCA ATG GCC CGG TCC TTT TCT TTA CTG ATG GTC GTG CTG GTA CTC AGC TAC AAA TCC ATC TGC TCT

     S23  1
  Leu Gly Cys Asp Leu Pro Gln Thr His Ser Leu Arg Asn Arg Arg Ala Leu Ile Leu Leu Ala Gln Met Gly Arg
  CTG GGC TGT GAT CTG CCT CAG ACC CAC AGC CTG CGT AAT AGG AGG GCC TTG ATA CTC CTG GCA CAA ATG GGA AGA
                                                    100
     25
  Ile Ser Pro Phe Ser Cys Leu Lys Asp Arg His Glu Phe Arg Phe Pro Glu Glu Glu Phe Asp Gly His Gln Phe
  ATC TCT CCT TTC TCC TGC TTG AAG GAC AGA CAT GAA TTC AGA TTC CCA GAG GAG GAG TTT GAT GGC CAC CAG TTC
                                                                            200
     50
  Gln Lys Thr Gln Ala Ile Ser Val Leu His Glu Met Ile Gln Gln Thr Phe Asn Leu Phe Ser Thr Glu Asp Ser
  CAG AAG ACT CAA GCC ATC TCT GTC CTC CAT GAG ATG ATC CAG CAG ACC TTC AAT CTC TTC AGC ACA GAG GAC TCA
     75
  Ser Ala Ala Trp Glu Gln Ser Leu Leu Glu Lys Phe Ser Thr Glu Leu Tyr Gln Gln Leu Asn Asp Leu Glu Ala
  TCT GCT GCT TGG GAA CAG AGC CTC CTA GAA AAA TTT TCC ACT GAA CTT TAC CAG CAA CTG AAT GAC CTG GAA GCA
                300
     100
  Cys Val Ile Gln Glu Val Gly Val Glu Glu Thr Pro Leu Met Asn Glu Asp Phe Ile Leu Ala Val Arg Lys Tyr
  TGT GTG ATA CAG GAG GTT GGG GTG GAA GAG ACT CCC CTG ATG AAT GAG GAC TTC ATC CTG GCT GTG AGG AAA TAC
                                                    400
     125
  Phe Gln Arg Ile Thr Leu Tyr Leu Met Glu Lys Lys Tyr Ser Pro Cys Ala Trp Glu Val Val Arg Ala Glu Ile
  TTC CAA AGA ATC ACT CTT TAT CTA ATG GAG AAG AAA TAC AGC CCT TGT GCC TGG GAG GTT GTC AGA GCA GAA ATC
                                                                            500
     150                                                            166
  Met Arg Ser Phe Ser Phe Ser Thr Asn Leu Gln Lys Arg Leu Arg Arg Lys Asp END
  ATG AGA TCC TTC TCT TTT TCA ACA AAC TTG CAA AAA AGA TTA AGG AGG AAG GAT TGA AAA CTG GTT CAT CAT GGA


  AAT GAT TCT CAT TGA CTA ATG CAT CAT CTC ACA CTT TCA TGA GTT CTT CCA TTT CAA AGA CTC ACT TCT ATA ACC
              600

  ACC ACA AGT TGA ATC AAA ATT TCC AAA TGT TTT CAG GAG TGT TAA GAA GCA TCG TGT TTA CCT GTG CAG GCA CTA
                                                    700

  GTC CTT TAC AGA TGA CCA
```

Figure 2

RAN 4100/19

HUMAN RECOMBINANT LEUKOCYTE L INTERFERON (IFLrL)

ATC ATA AAG AAA GCA AAA ACA GAG ATA GAA AGT AAA ACT AGG CAT TTA GAA AAT GGA AAT TAG TAT GTT CAC TAT
-100

TTA AGA CCT ATG CAC AGA GCA AAG TCT TCA GAA AAC CAT AGG CCG AAG TTC AAG GTT ATC CAT CTC AAG TAG CCT

```
                           S1
                           Met Ala Leu Ser Phe Ser Leu Leu Met Ala Val Leu Val Leu Ser Tyr Lys Ser
AGC AAT ATT TGC AAC ATC CCA ATG GCC CTG TCC TTT TCT TTA CTT ATG GCC GTG CTG GTG CTC AGC TAC AAA TCC
```

```
              S23  1
Ile END Ser Leu Gly Cys Asp Leu Pro Gln Thr His Thr Leu Arg Asn Arg Arg Ala Leu Ile Leu Leu Gly Gln
ATC TGA TCT CTG GGC TGT GAT CTG CCT CAG ACC CAC ACC CTG CGT AAT AGG AGG GCC TTG ATA CTC CTG GGA CAA
                                                          100
```

```
              25
Met Gly Arg Ile Ser Pro Phe Ser Cys Leu Lys Asp Arg His Asp Phe Arg Ile Pro Gln Glu Glu Phe Asp Gly
ATG GGA AGA ATC TCT CCT TTC TCC TGC CTG AAG GAC AGA CAT GAT TTC CGA ATC CCC CAG GAG GAG TTT GAT GGC
                                                                                            200
```

```
              50
Asn Gln Phe Gln Lys Ala Gln Ala Ile Ser Val Leu His Glu Met Ile Gln Gln Thr Phe Asn Leu Phe Ser Thr
AAC CAG TTC CAG AAG GCT CAA GCC ATC TCT GTC CTC CAT GAG ATG ATC CAG CAG ACC TTC AAT CTC TTC AGC ACA
```

```
              75
Glu Asp Ser Ser Ala Ala Trp Glu Gln Ser Leu Leu Glu Lys Phe Ser Thr Glu Ile Tyr Gln Gln Leu Asn Asp
GAG GAC TCA TCT GCT GCT TGG GAA CAG AGC CTC CTA GAA AAA TTT TCC ACT GAA ATT TAC CAG CAA CTG AAT GAC
                    300
```

```
              100
Leu Glu Ala Cys Val Ile Gln Glu Val Gly Val Glu Glu Thr Pro Leu Met Asn Glu Asp Ser Ile Leu Ala Val
CTG GAA GCA TGT GTG ATA CAG GAG GTT GGG GTG GAA GAG ACT CCC CTG ATG AAT GAG GAC TCC ATC CTG GCT GTG
                                                          400
```

```
              125
Arg Lys Tyr Phe Gln Arg Ile Thr Leu Tyr Leu Ile Glu Arg Lys Tyr Ser Pro Cys Ala Trp Glu Val Val Arg
AGG AAA TAC TTC CAA AGA ATC ACT CTT TAT CTA ATA GAG AGG AAA TAC AGC CCT TGT GCC TGG GAG GTT GTC AGA
                                                                                            500
```

```
              150                                                       166
Ala Glu Ile Met Arg Ser Leu Ser Phe Ser Thr Asn Leu Gln Lys Arg Leu Arg Arg Lys Asp END
GCA GAA ATC ATG AGA TCC CTC TCG TTT TCA ACA AAC TTG CAA AAA AGA TTA AGG AGG AAG GAT TGA AAA CTG GTT
```

CAA CAT GGC AAT GAT CCT GAT TGA CTA ATA CAT TAT CTC ACA CTT TCA TGA GTT CTT CCA TTT CAA AGA CTC ACT
600

TCT ATA ACC ACG ACG TGT TGA ATC AAA ATT TTC AAA TGT TTT CAG CAG TGT AAA GAA GTG TCG TGT ATA CCT GTG
700

CAG GCA CTA GTC CTT TAC AGA TGA CCA TTC TGA TGT CTC TGT TCA TCT TTT GTT TAA ATA TTT ATT TAA TTA TTT
800

TTA AAA TTT ATG TAA TAT CAT GAG TCG CTT TAC ATT GTG GTT AAT GTA CAA TAT TGT TCT TCA ATA TTT GCC AAT

ATA TT


Figure 3

0072541

Fragment from pBR322

Figure 4

Fragment from pBR322

Figure 5

RAN 4100/19